# EUROPEAN PATENT SPECIFICATION

(11) **EP 2 427 127 B1**
(45) Date of publication and mention of the grant of the patent: **28.02.2018**
(21) Application number: 10721317.5
(22) Date of filing: 10.05.2010
(51) Int. Cl.: A61B 17/06

(54) **ELASTOMERIC THREAD HAVING ANCHORING STRUCTURES FOR ANCHORING IN BIOLOGICAL TISSUES**
ELASTOMERER FADEN MIT VERANKERUNGSMERKMALEN ZUR VERANKERUNG IN BIOLOGISCHEN GEWEBEN
FIL ÉLASTOMÈRE DOTÉ DE STRUCTURES D'ANCRAGE POUR ANCRAGE DANS DES TISSUS BIOLOGIQUES

(30) Priority: 08.05.2009 DE 102009020894
(43) Date of publication of application: 14.03.2012
(73) Proprietor: Aesculap AG, 78532 Tuttlingen/Donau (DE); ITV Denkendorf Produktservice GmbH, 73770 Denkendorf (DE)
(72) Inventor: ODERMATT, Erich, 8200 Schaffhausen (CH); BERNDT, lngo, 78532 Tuttlingen (DE); KÖNIG, Silke, 78628 Rottweil (DE); OBERHOFFNER, Sven, 71384 Weinstadt-Endersbach (DE); MÜLLER, Erhard, 70565 Stuttgart (DE)
(74) Representative: Patentanwälte Ruff, Wilhelm, Beier, Dauster & Partner mbB
(86) International application number: PCT/EP2010/002848
(87) International publication number: WO 2010/127875

(56) References cited:
- EP-A1- 2 204 145
- WO-A1-2007/111407
- US-A- 5 222 976
- US-A- 5 713 920
- US-A1- 2004 060 409
- US-A1- 2005 267 532
- US-A1- 2007 224 237
- US-A1- 2009 248 066

## Description

The present invention relates to an elastomeric thread, to a wound closure system based thereon, to a surgical kit of parts, and to a process for producing the elastomeric thread. The standard way of closing wounds in surgery is by using thread-shaped sutures. These are typically knotted to achieve secure fixation in the tissue. Here care must be taken to ensure that the wounds to be closed are stitched together using an optimal force at the wound edges. If, for example, the wound edges are stitched together too loosely and too nonuniformly, there is a risk in principle of increased scarring or de-hiscence. If, by contrast, the wound edges are stitched together overly tautly, there is a risk that blood flow through the wound edges is restricted, which can give rise to necrotic changes in the surrounding tissue region.

In addition to the risk of possible secondary complications, which may necessitate renewed surgical interventions, there is also always a certain risk that wound closures based on knotted sutures will lead to disruptions in the healing process and more particularly to unsatisfactory cosmeses for the patient concerned. Another factor is that it is often necessary for several knots, more particularly up to seven knots, to be placed on top of each other to ensure a secure knotted hold. This means that there is a lot of material being introduced into the region of the wound to be cared for, and can lead to increased foreign-body reactions, more particularly in the case of absorbable sutures. Furthermore, the use of conventional sutures can also be of disadvantage in the management of difficult-to-access fields of indication, as typically occur in laparoscopy.

Sutures which, in contradistinction to known or conventional threads, do not have to be knotted have already been known for some time under the designation "barbed sutures". Such knotless or self-locking/self-fixating/self-retaining sutures usually consist of a monofil thread that has barbs along its longitudinal axis. Corresponding sutures are described for example in the printed publications US 3,123,077 A, EP 1 559 266 B1, EP 1 560 683 B1 and EP 1 556 946 B1. The barbs have generally been formed on the thread surface such that the thread can be pulled through the tissue in the direction of the barbs without great resistance and more particularly without significant tissue trauma. When pulled in the opposite direction, however, the barbs deploy and anchor themselves and hence also the suture in a surrounding tissue region. This prevents the suture being pulled back through a puncture channel formed by the suture.

Document US 2004/060409 A1 discloses self-retaining elastomeric sutures including tissue retainers, according to the preamble of independent claim 1. It is a profound disadvantage that, as the knotless or self-fixating sutures described in the previous section are introduced into tissue, the barbs, which generally protrude from the thread surface, can give rise to additional irritation of the tissue. In addition, as well as tissue traumatisation, the protruding barbs give rise to a larger puncture channel in the course of being pulled through the tissue. To avoid additional irritation or traumatisation of tissue, therefore, appropriate auxiliary means are frequently employed. For instance, techniques are known from facial surgery where initially a thin tube is led through the tissue to be treated before a barbed suture is led through this tube. After correct placement of the suture, the tube is removed, which causes the barbs to become erect and hence able to effect an anchoring of the suture in the surrounding tissue region. There are other surgical methods where a barbed suture is pulled together with a flexible tube surrounding the suture through a tissue to be treated. Once the sheathed suture is correctly placed, the flexible tube is pulled off, making it possible for the suture to become anchored via its barbs. Appropriate auxiliary means and suture systems based thereon are described for example in the printed publications US 6,241,747 B1 and DE 10 2005 004 318 A1. The use of additional auxiliary means makes the above-described methods more costly, inconvenient and complicated to carry out and thereby raises the likeli-hood of mistakes being associated therewith.

It is an object of the present invention to provide an ideally atraumatic, knotless or self-fixating wound closure system which in respect of its construction and more particularly its handling corresponds in principle to a surgical suture yet does not require additional auxiliary means for avoiding tissue trauma on introducing the wound closure system into a biological tissue.

We have found that this object is achieved by an elastomeric thread having the features of independent claim 1. Preferred embodiments of the thread of the present invention are subject matter of dependent claims 2 to 12. A further aspect of the present invention relates to a wound closure system having the features as per independent claim 13. The present invention further also encompasses a surgical kit of parts having the features of independent claim 14. An additional aspect of the present invention relates to a process for producing the thread of the present invention as per independent claim 15. The wording of all the claims is hereby incorporated in this description by reference.

The thread of the present invention comprises an elastomeric thread, preferably for use as a knotless or self-fixating wound closure system, more particularly in the manner of a knotless or self-fixating surgical suture, wherein the thread includes on its surface anchoring structures for anchoring in biological, more particularly human and/or animal, tissues. In other words, the invention provides a thread based on an elastomeric material, generally a polymer having elastomeric or elastic properties (a so-called elastomer), wherein anchoring structures are formed on the thread surface for anchoring the thread in biological tissues.

The biological tissues may in principle comprise hard and/or soft tissues. The tissues are preferably selected from the group consisting of skin, fat, fascia, bone, muscle, organs, nerves, blood vessels, connective tissue, sinews, tendons and ligaments. The thread of the present invention is generally useful for tissue fixation and/or tissue lifting. The elastomeric thread is more particularly suitable for applications in plastic surgery, preferably for tightening the skin. For example, the elastomeric thread is suitable for performing an eyebrow lift. Further fields of application are cheek and/or jaw line corrections. In addition, however, the thread of the present invention is also suitable for other surgical indications, more particularly for indications where the use of conventional sutures is made difficult by steric hindrance for example. The elastomeric thread is thus particularly useful for performing laparoscopic interventions. A further field of use concerns the fixation of implants, more particularly textile implants. Examples of textile implants indude hernia, prolapse and urinary incontinence meshes. A further possible field of application for the thread of the present invention relates to the performance of anastomoses, in particular vascular or intestinal anastomoses.

In the elastomeric thread of the present invention, the anchoring structures absent an axial pulling load on the thread (relaxed or nonelongated state of the thread) essentially do not protrude from the thread surface, so that the thread has an essentially smooth surface. As a result, tissue traumas on pulling the thread through a biological tissue can be substantially avoided. The expression "essentially smooth" as to be understood herein includes minor elevations on the thread surface which are capable of resulting from a position of the anchoring structures close to the thread surface in the nonelongated state of the thread. Ideally, however, the thread surface is completely smooth in the relaxed or nonelongated state of the thread.

The anchoring structures themselves are erectable, more particularly reversibly erectable. The anchoring structures are erectable on imposition of an axial pulling load on the thread, i.e. by applying a pulling force in the longitudinal direction of the thread, preferably at its ends. When the thread is extended or elongated by application of an axial pulling force, the anchoring structures become erect with particular advantage. The anchoring structures become erect because the force applied, although extending the thread by simultaneously thinning the thread diameter, does not transmit, or transmits at least only partially, to the anchoring structures. On removal of the pulling force, the thread relaxes, i.e. shortens, again as a consequence of the elastic restoring force, whereby the anchoring structures anchor themselves in the tissue and can for example approximate wound edges or tighten skin in cosmetic surgery.

Preferably, the anchoring structures in an erected state form an angle between 10 and 80°, more particularly 15 and 70° and preferably 20 and 60°, with the surface of the thread. In a further embodiment, the anchoring structures absent an axial pulling load on the thread, i.e. in the relaxed state of the thread, are formed on the thread surface in a density between 1 and 30 and more particularly 4 and 20 per cm of thread length (projected in intervals onto the longitudinal axis of the thread).

The thread of the present invention comprises in general a thread main body, on the surface of which the anchoring structures for anchoring in biological, more particularly human and/or animal, tissues are formed. The thread main body usually has an elongate form. Preferably, the thread main body and the anchoring structures are formed in one piece. In principle, the thread main body and the anchoring structures can be formed from different elastomeric materials, more particularly elastomers. Preferably, the anchoring structures are formed from the same elastomeric material, more particularly the same elastomer, as the thread main body. In one possible embodiment, the thread main body is formed from an elastomeric material, more particularly an elastomer, and the anchoring structures are formed from a nonelastomeric material, more particularly nonelastomeric polymer. With regard to possible elastomeric materials, more particularly elastomers, the following description is ref erenced in full.

The thread of the present invention can in principle be made of all elastomeric materials suitable therefor. Preferably, the thread material comprises a polymer having elastomeric properties, i.e. an elastomer. The polymer can be a homo-, co-, ter- or tetrapolymer and so on. In what follows, the term "copolymers" is to be understood as generally meaning polymers composed of two or more different monomeric units. In other words, copolymers useful for the purposes of the present invention may also comprise terpolymers, tetrapolymers, and so on. Suitable elastomers are more particularly formed as block polymers or segmented polymers. Formation as random or adventitious or alternating co-, ter- or tetrapolymers and so on is similarly possible according to the present invention. It is particularly preferable according to the present invention when the thread is formed from a block copolymer, block terpolymer, segmented polymer or a polymer blend. The block co- or block terpolymer may more particularly comprise a multiblock copolymer and a multiblock terpolymer, respectively. More particularly, the thread may be formed from a thermoplastic elastomer. Thermoplastic elastomers generally comprise plastics which at room temperature behave comparably to the classic elastomers, but on heating are plastically deformable and thus exhibit a thermoplastic behaviour. Ideally, thermoplastic elastomers useful according to the present invention have a transition temperature to the plastically deformed behaviour which is sufficiently above the body temperature of a patient. The use of thermoplastic elastomers has the advantage that they are significantly better to process than the classic elastomers. Classic elastomers are generally chemically wide-meshedly crosslinked spatial net molecules wherein the crosslinks can generally not be broken without decomposition of the elastomeric material. By contrast, thermoplastic elastomers include subregions of physical points of crosslinking, which can be broken on heating without the thermoplastic elastomeric material decomposing as a result.

In an advanced embodiment, the elastomeric thread is formed from nonabsorbable, partially absorbable or fully absorbable elastomers. Nonabsorbable elastomers are preferably selected from the group consisting of thermoplastic elastomers based on olefin, thermoplastic elastomers based on urethane, or thermoplastic polyurethanes, thermoplastic copolyesters, styrene block copolymers, thermoplastic copolyamides, silicone elastomers, copolymers thereof and mixtures thereof. For example, the thread can be formed from a nonabsorbable elastomer from the group consisting of acrylic rubber, polyester-urethane rubber, brominated butyl rubber, chlorinated butyl rubber, chlorinated polyethylene, epichlorohydrin homopolymer, polychloroprene, sulphonated polyethylene, ethylene-acrylate rubber, epichlorohydrin copolymer, sulphur-crosslinked ethylene-propylene-diene rubber, peroxidically crosslinked ethylene-propylene copolymer, polyether-urethane rubber, ethylene-vinyl acetate copolymer, fluororubber, fluorosilicone rubber, hydrogenated nitrile rubber, butyl rubber, vinyl-containing dimethylpolysiloxane, nitrile rubber, butadiene-acrylonitrile rubber, natural rubber, thioplasts, polyfluorophosphazenes, polynorbonene, styrene-butadiene rubber, carboxyl-containing nitrile rubber, copolymers thereof and mixtures thereof. The thermoplastic polyurethanes mentioned in this section may in principle comprise aliphatic or aromatic polyurethanes. The polyurethanes may more particularly be branched or unbranched. According to the present invention, unbranched aliphatic thermoplastic polyurethanes are preferred. In an advanced embodiment, the thermoplastic polyurethanes are selected from the group consisting of aliphatic polycarbonate urethanes, aromatic polycarbonate urethanes, silicone polycarbonate urethanes, silicone polyether urethanes and combinations thereof. The polycarbonate urethanes are generally prepared starting from a polycarbonate having terminal hydroxyl groups, an aromatic diisocyanate and also an oligoglycol or polyglycol.

In a further embodiment, the elastomeric thread is composed of absorbable elastomers selected from the group consisting of polyhydroxybutyrates, block copolymers thereof, block terpolymers thereof and combinations, more particularly blends, thereof. Suitable block copolymers comprise glycolide, lactide, ε-caprolactone, trimethylene carbonate, p-dioxanone, 3-hydroxybutyrate and/or 4-hydroxybutyrate. Advantageous block terpolymers comprise glycolide, lactide, ε-caprolactone, trimethylene carbonate, p-dioxanone, 3-hydroxybutyrate and/or 4-hydroxybutyrate. For example, the thread of the present invention can be formed from a triblock terpolymer comprising glycolide, trimethylene carbonate and ε-caprolactone. Such a triblock terpolymer is commercially available for example under the name of Monosyn®. The aforementioned polyhydroxybutyrates preferably comprise poly-3-hydroxybutyrate and/or poly-4-hydroxybutyrate.

In a further embodiment, the anchoring structures and the thread surface are formed in one piece. The anchoring structures are preferably formed from the elastomeric thread material. The anchoring structures are preferably formed as cuts into the surface of the elastomeric thread. The cuts may comprise mechanical, physico-chemical, more particularly laser-produced, or thermal cuts. The cuts may have a cut depth, measured perpendicularly from the thread surface, between 5 and 60%, more particularly 15 and 50%, preferably 20 and 40%, based on the diameter of the thread.

In a further embodiment, the anchoring structures on imposition of an axial pulling load on the thread form a barb, escutcheon, shield, scale, wedge, spike, arrow, V and/or W shape. It is particularly preferable according to the present invention for the anchoring structures to be designed in the manner of barbs or as barbs.

The anchoring structures may on imposition of an axial pulling load on the thread be in principle formed in different arrangements on the thread surface. For example, the anchoring structures may in this case have a row-shaped arrangement, a staggered arrangement, a zigzag-shaped arrangement, a spiral-shaped arrangement, a helical-shaped arrangement, a random arrangement or combinations thereof in the longitudinal and/or transverse direction, preferably in the longitudinal direction, of the thread.

In a further embodiment, the elastomeric thread on imposition of an axial pulling load includes at least one set, more particularly two, three or more sets, of anchoring structures. A set of anchoring structures is herein to be understood as meaning an arrangement of anchoring structures on the thread which in respect of the configuration of the anchoring structures, for example length of the anchoring structures, height of the anchoring structures, cut depth of the anchoring structures, angle of the anchoring structures which the anchoring structures form in the erected state with the thread surface, orientation of the anchoring structures and/or shape or form of the anchoring structures coincides.

In a possible embodiment, the anchoring structures on imposition of an axial pulling load on the thread form a unidirectional arrangement on the surface of the thread. In other words, the anchoring structures in this embodiment point uniformly in one direction.

In a particularly preferred embodiment, the elastomeric thread on imposition of an axial pulling load has a so-called bidirectional arrangement of the anchoring structures on the thread surface. A bidirectional arrangement of anchoring structures is herein to be understood as meaning an arrangement wherein the anchoring structures are formed in two different directions (bidirectionally) on the thread surface. Preferably, viewed in the longitudinal direction of the thread, the anchoring structures point for a first thread portion in the direction of a remaining second thread portion and for the remaining second thread portion point in the direction of the first thread portion. For example, viewed in the longitudinal direction of the thread, the anchoring structures for a first thread portion may be formed in the direction of the thread middle and for a remaining second thread portion may likewise be formed in the direction of thread middle. Here the length of the thread portions may correspond approximately to half the thread length.

In an advanced embodiment, the elastomeric thread on imposition of an axial pulling load displays on its surface at least two bidirectional arrangements of anchoring structures. It is particularly preferable when, relative to a first bidirectional arrangement of anchoring structures, a second bidirectional arrangement of anchoring structures is formed on the thread surface in the circumferential direction of the thread by about 180° and preferably offset to the first bidirectional arrangement. It can further be provided according to the present invention for the elastomeric thread to display altogether three bidirectional arrangements of anchoring structures on imposition of an axial pulling load. It is preferable in this case when, relative to a first bidirectional arrangement of anchoring structures, a second bidirectional arrangement of anchoring structures is formed on the thread surface in the circumferential direction of the thread by about 120° and preferably offset to the first bidirectional arrangement, and this second bidirectional arrangement of anchoring structures being in turn formed in the circumferential direction of the thread by about 120° and preferably offset relative to a third bidirectional arrangement of anchoring structures, so that the third bidirectional arrangement of anchoring structures is similarly formed in the circumferential direction of the thread by about 120° and preferably offset relative to the first bidirectional arrangement of anchoring structures.

In a further preferred embodiment, the anchoring structures on imposition of an axial pulling load on the thread have a periodically changing, more particularly alternating, orientation on the thread surface. For example, viewed in the longitudinal direction of the thread, the anchoring structures for a first thread portion can be formed in the direction of a second thread portion and for the second thread portion in the direction of the first thread portion, for a third thread portion adjoining the second thread portion in the direction of a fourth thread portion and for the fourth thread portion in the direction of the third thread portion, and so on.

It can further be provided according to the present invention for the elastomeric thread to display areal regions or areal portions without anchoring structures. Preferably, the thread displays an areal portion without anchoring structures approximately in the region of the thread middle. This areal portion, viewed in the longitudinal direction of the thread, can have a length between 0.5 and 5 cm, more particularly 1.5 and 3 cm. This makes it possible to place the thread ends side by side to form a loop and to attach them to a surgical inserting instrument, preferably to a surgical needle. In this embodiment, the remaining areal portions of the thread preferably display a bidirectional arrangement of anchoring structures, so that, after loop formation, the anchoring structures point unidirectionally in the direction of the loop. Since the thread middle generally is used for the actual wound closure, a thread whose thread middle is essentially free of anchoring structures can be used with particular advantage to avoid additional irritations of tissue in the wound region. With regard to the arrangement possibilities for anchoring structures on the thread surface on imposition of an axial pulling load on the thread, reference is made to the description heretofore.

In a further embodiment, the anchoring structures include a coating, preferably a stiffening coating. The coating is preferably only formed in the region of the anchoring structures, more particularly exclusively on the anchoring structures. According to the present invention, the coating may only be formed partially on the anchoring structures. For example, the coating may only be formed on the reverse side of the anchoring structures. Furthermore, the coating may only be formed in the region of the ends protruding from the thread surface, preferably only on the tips, of the anchoring structures, more particularly on both sides thereof. However, it is preferable for the coating to surround the anchoring structures in full, i.e. for the surface of the anchoring structures to be completely covered with the coating. The coating preferably comprises a proportion between 0.5% and 25% by weight, more particularly 1% and 20% by weight and preferably 2% and 15% by weight, based on the total weight of the thread. In general, the layer thickness of the coating is chosen such that the coated anchoring structures still protrude from the thread surface. The layer thickness of the coating is preferably between 1 and 100 µm, more particularly 2 and 50 µm and more preferably 4 and 40 µm. In general, the coating is formed from a different material than the thread per se. Preferably, the coating includes a biocompatible material, more particularly polymer, having a flexural modulus > 1500 N/mm², preferably > 4000 N/mm². More particularly, the coating is formed from such a material, more particularly polymer.

The coating which is preferably a stiffening coating may further be formed to be fully absorbable, partially absorbable or nonabsorbable. More particularly, the stiffening coating may include an absorbable polymer and more particularly be formed from such. Useful polymers include in particular polyhydroxyalkanoates, copolymers thereof and combinations thereof. The coating preferably includes an absorbable polymer selected from the group consisting of polylactide, polyglycolide, poly-ε-caprolactone, polytrimethylene carbonate, poly-p-dioxanone, copolymers thereof and combinations, more particularly blends, thereof. Preferably the coating is formed from one of the polymers mentioned in this section, copolymers mentioned in this section or from one of the combinations mentioned in this section, more particularly blends, thereof.

In an alternative embodiment, the coating, preferably stiffening coating, includes a nonabsorbable polymer, more particularly selected from the group consisting of polyethylene terephthalate, polypropylene, polyvinylidene difluoride, polytetrafluoroethylene, polyhexafluoropropylene, polyamides, copolymers thereof and combinations, more particularly blends, thereof. Preferably, the coating is formed from a polymer or copolymer mentioned in this section or from one of the combinations, more particularly blends, mentioned in this section.

In a further embodiment, the thread has a linear tensile strength between 30 and 500 N/mm², more particularly 50 and 350 N/mm², based on a thread without anchoring structures. The thread of the present invention may further have an elongation at break between 50 and 700%, more particularly 80 and 600%, preferably 150 and 500%. The thread preferably has a flexural modulus of 3 to 300 N/mm², more particularly 5 to 200 N/mm².

In principle, the thread of the present invention can be formed as a monofilament or as a multifilament, more particularly as a braided or intertwined multifilament. Preferably, the elastomeric thread is formed as a monofilament or as a monofil thread. It is further possible according to the present invention for the thread to be formed as a pseudomonofilament.

In a further embodiment, the thread is designed as a mass or solid thread. As used here, the term "mass or solid thread" may in particular mean that the thread has no lumen.

In an alternative embodiment, the thread is designed as a hollow thread, in particular as a tubular thread, preferably as a tube or hose. The hollow thread may be in particular featured by a closed wall encasing a lumen, wherein the ends of the hollow thread are preferably open. The hollow thread may be produced by means of extrusion. Further, the hollow thread may have an internal diameter < 1 cm, in particular < 5 mm, preferably < 2 mm. The anchoring structures, on imposition of an axial pulling load on the hollow thread, may be arranged into the interior and/or exterior, preferably exterior, of the hollow thread. The anchoring structures may be cut into the wall of the hollow thread, wherein the cuts do not break through the wall of the hollow thread, and/or may be present as breakthroughs, i.e. the anchoring structures completely break through the wall of the hollow thread.

Hollow threads according to the present invention may be employed in various fields of medicine. For instance, the hollow threads may be used as a self-anchoring infusion tubes, delivery tubes, catheters, distribution systems for medicaments, in particular liquid medicaments, drug-release-systems or drainage systems, in particular drainage tubes. Besides, the hollow threads may be applied, in particular shrunk and/or drawn, on a round stock, fibre, monofilament, pseudomonofilament, multifilament, yarn, thread or the like in order to equip these structures with self-anchoring properties.

The thread according to the present invention generally has a circular cross section. However, other cross-sectional shapes are conceivable as well. For example, the thread may have an oval, triangular, trilobal, square, trapezoidal, rhomboid, pentagonal/five-cornered, hexagonal/six-cornered, star-shaped or cruciform cross section. Such cross-sectional shapes are readily realizable with the aid of appropriate extrusion dies which can be custom made to any desired cross-sectional shape.

In a further embodiment, the elastomeric thread includes added substances, more particularly biological and/or medical actives. For example, the thread may include antimicrobial, disinfecting, growth-promoting, anti-inflammatory, blood-coagulating, pain-killing (analgesic) and/or odour-controlling actives.

In a further embodiment, the elastomeric thread is present in the form of a continuous-filament thread, in particular continuous-filamenet fibre, or as a converted, more particularly cut to length, fibre.

In a further embodiment, the elastomeric thread is fitted at least at one end, preferably at both ends, with a surgical inserting instrument. The surgical inserting instrument preferably comprises a surgical needle. The use of a canula as inserting instrument is similarly possible according to the present invention. Preferably, at least one end of the canula has been sharpened with an oblique grind in order that a small cut be made on penetration into a tissue. Alternatively, the canula may also have a conically tapered and more particularly pointed end. In the case of the above-described loop-shaped thread, it is generally the case that both ends of the thread are attached to a surgical inserting instrument. However, when the thread has a bidirectional arrangement of anchoring structures, it is preferable according to the present invention when both of the ends of the thread are each connected to a surgical inserting instrument. To connect the thread to a surgical inserting instrument, the thread can be inserted into a dedicated drilled hole in the inserting instrument and the inserting instrument subsequently be compressed or beaded in the region of the drilled hole.

To avoid puncture channel bleeds, it can be further provided according to the present invention for the thread to have a smaller diameter in the region of its ends than in the remaining thread regions. In other words, the ends of the thread may have a tapered diameter. Such a thread is particularly advantageously combined with a surgical inserting instrument, for example a surgical needle, that is actually designed for a smaller thread diameter. In this way, the thread diameter can be conformed to the diameter of the inserting instrument. According to the present invention, especially a diameter ratio of surgical inserting instrument to thread of less than 2:1, preferably of 1:1, can be provided. As a result, a puncture channel formed by the surgical inserting instrument can be better filled out by the thread regions having the original, i.e. untapered, diameter. A puncture channel whose diameter is very close to the diameter of the thread is advantageous in ensuring better anchoring of the thread in the tissue. To taper the diameter, the thread can be shaved in the region of its ends. Thermal methods or laser techniques can be used for this for example. The transition from the original diameter of the thread to the tapered diameter in the region of the thread ends can be abrupt or continuous, more particularly in the form of a gradient. To form a gradual transition, extrusion technology is suitable in particular. The take-off speed involved in extruding a thread can be varied, more particularly periodically. This can be accomplished for example by modulating the circumferential speed of the godet responsible for the take-off of the thread. Alternatively, additional godets can be interposed between the extrusion die and the take-off godet.

In a particular embodiment, the thread is fixed in its lengthened or elongated state in the lumen of a blunt end of a canula. This results in a small ratio of outer diameter of the canula to diameter of the thread. Preferably, the canula is longer than the pull-through distance through the tissue. This can be realized for example by temporarily fixing the elastomeric thread in the elongated state (with tapered diameter) by freezing and then pushing it into the canula. As it thaws, the thread then becomes anchored in the canula as it shortens and its diameter increases, resulting in an optimal needle/thread diameter ratio.

In a further embodiment, the elastomeric thread is present in a drawn or undrawn state.

A further aspect of the present invention relates to a wound closure system or a surgical fixating means, more particularly a knotless or self-fixating wound closure system or surgical fixating means, preferably in the manner of a surgical suture, more particularly knotless or self-fixating surgical suture, comprising an elastomeric thread as per the present invention. The wound closure system is generally present as a thread-shaped wound closure system. As such, the wound closure system is preferably formed to be monofil. In principle, however, the wound closure system can also be formed to be multifil. In respect of further features and details concerning the wound closure system, reference is made to the description heretofore.

The present invention further provides a surgical kit of parts comprising an elastomeric thread or a wound closure system as per the present invention and at least a surgical inserting instrument. As mentioned, the surgical inserting instrument may comprise a surgical needle or a canula. With regard to further features and details concerning the kit of parts, reference is likewise made to the description heretofore.

The present invention further relates to a process for producing a thread of the present invention, wherein anchoring structures are formed on the surface of an elastomeric thread.

In a preferred embodiment, the anchoring structures are formed by cutting into the thread surface.

In an advanced embodiment, the anchoring structures are cut into the thread mechanically, preferably by means of at least one cutting blade, for example a microtome knife or, as an alternative, by means of a needle, in particular a surgical needle, in order to less affect the mechanical stability of the thread. Customary cutting devices can be used for this purpose. Useful cutting devices generally comprise a cutting board, at least one cutting blade and also holding or fixing elements for the thread, for example a vice, chucks, holding or clamping jaws. For example, cutting the anchoring elements mechanically may utilize a cutting board (cutting abutment) having a groove, the groove being intended to receive the thread to be cut into. Depending on the depth of the groove, the use of at least one cutting blade allows specific control of the cut depth to which the anchoring structures are cut into the thread. This is because the at least one cutting blade is generally configured such that with it only at most the regions of the thread which protrude from the groove can be cut into. This contributes with particular advantage to enhancing the cutting consistency in the process of the present invention.

In an alternative embodiment, the anchoring structures are cut into the thread thermally, preferably in a temperature range between 10 and 100°C, more particularly 20 and 50°C, above the melting point of the thread material. Thermal cutting of the anchoring structures has the profound advantage over mechanical cutting that the cut ends in the thread which are produced by thermal cutting are less tapered, more particularly less acute, than results from purely mechanical cutting. This can be used to minimize the risk of the thread developing, under load, a tear starting from the respective cut ends. In an advanced embodiment, the anchoring structures are cut into the thread by means of a cutting wire, more particularly metal wire, suitable for this purpose. A heated, more particularly an electrically heated, cutting wire can be used for example. The cutting wire preferably comprises a fine wire. Preference is given to using a cutting wire having a diameter between 20 and 50 µm. As an alternative to a single cutting wire, it is also possible to use a sheet of cutting wires. It is similarly possible according to the present invention to use a metal grid.

In a further alternative embodiment, the anchoring structures are cut into the thread by means of laser cutting techniques. Useful lasers include in principle not only gas lasers, for example CO₂ lasers, but also solid state lasers, for example Nd:YAG lasers. In general, a suitable laser cutting machine consists of a laser beam source, a beam guide and a usually mobile system of focusing optics (concave mirror or positive lens). The beam leaving the beam source is guided either through an optical fibre in the case of an Nd:YAG laser for example, or via a deflecting mirror in the case of a CO₂ laser for example, to the machining optics which focus the laser beam and thereby produce the power densities required for cutting, which generally range from 10⁶ to 10⁹ W/cm². Appropriate laser cutting processes are well known to a person skilled in the art, so that more far-reaching observations can be dispensed with here.

In a particularly preferred embodiment, the anchoring structures are coated with a material, preferably a stiffening material, more particularly a stiffening polymer. Preferably, the thread is extended/elongated by application of an axial pulling force, preferably at the thread ends, to coat the anchoring structures. Here the anchoring structures, which are generally not subject to any significant extension or elongation, are preferably completely coated with the material and thereby stiffened. According to the present invention, the anchoring structures can be coated by dipping into a coating solution or by deposition of a material from the gas phase. As a result, relatively long anchoring structures, which in contradistinction to relatively short anchoring structures generally have a higher flexural slackness, can be stiffened in particular, whereby their anchoring performance can be improved overall. It is particularly preferable when the thread is alternatingly elongated (extended) and relaxed until the stiffening material has cured. This measure makes it possible to prevent the elastic properties of the thread main body (thread body without the barbs) being impaired or lost entirely.

The cutting of the anchoring structures can be effected in the drawn or undrawn state of the thread. Any drawing is preferably effected while heat is applied. To produce a heat advantageous for the drawing operation, warm water or infrared radiation can be used for example. It is similarly possible according to the present invention for drawing to be carried out in a heating oven suitable for this purpose. To draw the thread, it is usually guided over a roller or godet system comprising a set of rollers or godets which can have different speeds of rotation. Generally, each subsequent roller has a higher speed of rotation than the preceding roller of the drawing system. However, alternatively to the just-described continuous drawing, it is also possible according to the present invention to carry out discontinuous drawing. For discontinuous drawing, the thread can be clamped between the clamping jaws of a clamping device and subsequently drawn.

After drawing, optionally also independently of any drawing, of the thread it can be envisaged according to the present invention for the thread to be subjected to various post-treatment steps. In general, the thread is for this heat-conditioned in vacuo or at reduced pressure. This can be used to raise the crystallinity of the thread and, more particularly, lower the residual monomer content. A further advantage which can be achieved through a post-treatment of the thread relates to the reduced susceptibility to shrinking.

In order to minimize the righting angle of the anchoring structures, the thread may be subjected to a relaxation, in particular shrinkage, subsequent to a drawing. This is particularly advantageous with respect to polymers having a high drawing ratio. Such polymers may otherwise contribute to anchoring structures which may protrude perpendicularly from the thread surface on imposition of an axial pulling load on the thread. Further, due to a relaxation, elasticity of the thread may be improved which is advantageous in view of handling properties and a more equal transfer of force on a tissue to be treated.

In a further embodiment, the thread may be subjected to a relaxation, in particular shrinkage. Subsequently, the anchoring structures are cut into the relaxed thread. Thereafter, the cut-in thread is drawn. Thus, the righting angle of the anchoring structures may also be minimized.

The present invention further provides for the use of the thread of the present invention for producing a wound closure system or surgical fixating means, more particularly a knotless or self-fixating wound closure system or fixating means, preferably in the manner of a surgical suture, more particularly in the manner of a knotless or self-fixating surgical suture. The wound closure system or surgical fixating means is preferably useful for tissue fixation or tissue lifting. With regard to further features and details, the description heretofore is referred to in its entirety.

A further aspect of the present invention encompasses the use of the thread of the present invention in the production of a fixating means for implants, more particularly textile implants. With regard to further features and details, the description heretofore is similarly referenced in full.

Finally, the present disclosure also relates to an exemplary process for approximating, fixating and/or gathering together of biological, more particularly human and/or animal, tissue, comprising the steps of:
a) introducing into the tissue an elastomeric thread displaying on its surface anchoring structures for anchoring in biological, more particularly human and/or animal, tissues,
b) applying an axial pulling force to the inserted thread, more particularly at its ends, and
c) reducing, more particularly removing, the axial pulling force.

The elastomeric thread according to the present invention is particularly useful as a knotless or self-fixating wound closure system or surgical fixating means by displaying anchoring structures formed in an elastomeric thread material. When the thread is in a relaxed, i.e. nonextended, state, the anchoring structures combine with the remaining regions of the thread to form a smooth surface for the thread.

When the thread is then pulled through a biological, generally human or animal, tissue, this is preferably done by applying a force smaller than the force needed to erect the anchoring structures. This is particularly advantageous in completely avoiding tissue traumas. If desired, however, it can also be envisaged according to the present invention for the thread to be inserted into a biological tissue using a force which leads to minimal erection or deployment of the anchoring structures. The tissue traumas caused by this are then still significantly less than when the thread is introduced into a biological tissue with the anchoring structures maximally erected or deployed. Once the thread is optimally placed in the eyes of the surgeon, the anchoring structures are erected by exerting a pulling force on the two ends of the thread. After removal of the pulling force from the ends of the thread, the thread shortens, and the anchoring structures anchor themselves and hence the thread in the tissue. Since, however, the thread does not shorten to its original length and therefore is still under a certain tension, the tissue is, more particularly, the wound edges are, approximated, fixated and/or gathered together. Since the anchoring structures only erect on imposition of a certain pulling load on the thread in its longitudinal direction (axial pulling load), no additional aids are needed to introduce the thread into a biological tissue. This renders the handling of the thread of the present invention simple and free of complications. It is a further advantage of the thread of the present invention that it can in practice be used as a knotless or self-fixating surgical suture. Since surgeons are sufficiently familiar with such sutures, this results in a further advantage for the practical handling of the thread of the present invention.

Further features of the invention become apparent from the following description of preferred embodiments by reference to examples and a figure description in conjunction with the features of the subclaims and the figure. Individual features here can each be actualized on their own or two or more at a time in combination with each other, according to the appended claims.

### 1. Figure description

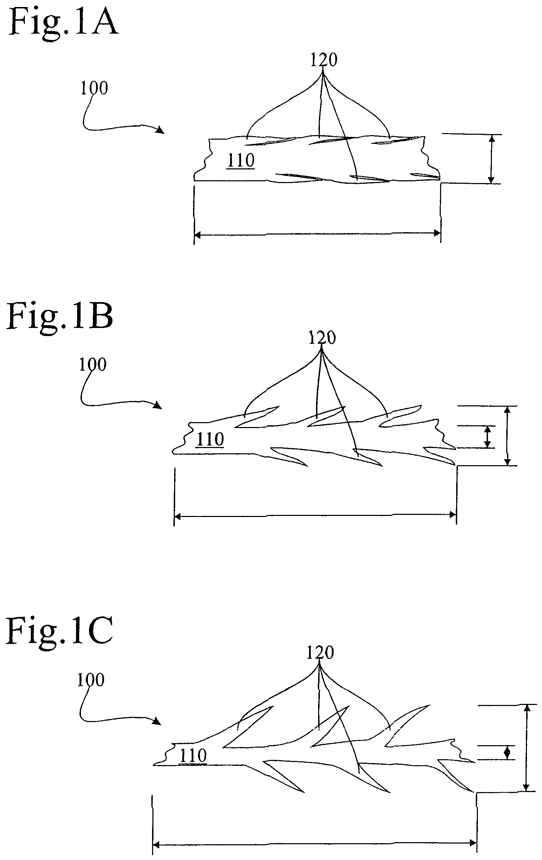

The single figure depicts a preferred embodiment of an inventive thread 100. The thread 100 has a thread main body 110 having anchoring structures 120 formed on its surface. In the relaxed (nonextended or nonelongated) state, the anchoring structures 120 combine with the thread main body 110 to form a smooth surface for the thread (fig. 1A). In this state, the thread can be pulled with particular advantage through a biological tissue without significant tissue traumas occurring as a result. Advantageously, the thread can also be moved in a biological tissue in a direction contrary to the potential blocking direction. When the thread 100 is subject to the imposition thereon of a certain pulling force in the longitudinal direction of the thread 100, preferably on the thread's ends, the anchoring structures 120 become erect (figures 1B and 1C). The application of the pulling force reduces the diameter of the thread main body. However, the pulling force is transmitted only partially, if at all, to the anchoring structures 120. When the thread 100, therefore, is used as a surgical suture, the anchoring structures 120 - after correct placement of the thread 100 in a biological tissue - can be erected by applying an axial pulling force to thereby establish contact with the surrounding region of tissue. Removing the axial pulling force causes the anchoring structures to become anchored in the biological tissue, since the anchoring structures are by virtue of the lengthwise contraction of the thread (due to the removal of the axial pulling force) pulled laterally of the thread 100 into the tissue. The elastic pull stops the anchoring structures becoming free again. As a result, the tissue can be approximated, fixated and/or gathered together as desired. In other words, an active approximation, fixation and/or gathering together of the tissue rests on the elastic restoring force of the thread 100.

### 2. Examples

### 2.1 Production of monofilaments from a thermoplastic elastomer (Vasomer)

Vasomer is a linear aliphatic polyurethane and a thermoplastic elastomer which is approved for medical implants and has a Shore A hardness of 76. Vasomer was processed into monofilaments on a TW 100 extruder (twin-screw extruder with Rheocord 90 drive from Haake). The cooled feed zone was followed at the extruder zones by a temperature profile rising from 160°C for zone 1 to 200°C at the spin head. The spin head was equipped with a 1.25 mm die with UD 8. Mass throughput was merely set via the screw speed. To solidify the extruded strand following an air passage of 7 cm it was pulled through a cold water bath at 20°C and subsequently wound up on a drum. Altogether, monofilaments were produced in two sizes. These are reproduced below in table 1.

**Table 1**

| Monofilament | MV1 | MV2 |
|---|---|---|
| Screw speed [rpm] | 24 | 22 |
| Die pressure [bar] | 45 | 40 |
| Winding speed [m/min] | 13.0 | 6.4 |
| Diameter [mm] | 0.80 | 1.05 |

The flexural modulus of the monofilaments, as measured on a Frank 58963 flexural stiffness tester at a clamped length of 5 mm and a bending angle of 30°, was merely 6 N/mm².

Stress-strain measurements and also hysteresis tests concerning reversible elasticity were carried out on a Zwick 1455 tensile tester. The maximum force for MV2 was found to be 39.1 N. The related extension was 410% ± 10%. Since the original diameter was approximately halved at elongation around 400%, strengths close to the USP requirements for surgical sutures were obtained. Hysteresis tests at 200% and 300% showed virtually complete reversibility.

### 2.2 Production of a Vasomer monofilament with barbs

The cutting of MV2 from example 2.1 was done on a specially designed prototype cutting machine. The cutting device used was a microtome knife, which cut into the MV2 monofilament pneumatically and at controllable time intervals. The cutting abutment and transporter used was a cutting wheel which was equipped with a fine guiding groove and onto which the Vasomer monofilament was clamped or adhered under most minimal elongation. The cutting wheel itself was driven at a controllable speed. The cut length, the cut angle and the resulting cut depth (perpendicularly to the monofilament surface) were likewise able to be varied. The results reproduced below in table 2 were obtained.

**Table 2**

| **Sample** | Vas B1 | Vas B2 | Vas B3 | Vas B4 | Vas B5 | Vas B6 |
|---|---|---|---|---|---|---|
| **State** | elastic, nonelongated | elastic, nonelongated | elastic, nonelongated | elastic, nonelongated | elastic, nonelongated | elastic, nonelongated |
| **Blade** | Microtome | Microtome | Microtome | Microtome | Microtome | Microtome |
| **Cut length [mm]** | 0.2 | 0.45 | 0.7 | 1.05 | 0.3 | 0.7 |
| **Perpendicular cut depth [mm]** | 0.07 | 0.15 | 0.24 | 0.36 | 0.15 | 0.35 |
| **Angle [°]** | 20 | 20 | 20 | 20 | 30 | 30 |

In the nonelongated state (even after repeated reversible elongation), the cuts were virtually not noticeable. The barbs in this state thus did not protrude from the monofilament surface. On the contrary, the barbs only deployed on elongation due to a pulling load in the longitudinal direction of the monofilament. After removal of the pulling tension and the return of the monofilament in its original state, the barbs became fully closed again and combined with the monofilament main body to form a completely smooth surface.

These tests document that the thread of the present invention is very useful to be inserted virtually atraumatically into a biological tissue using a suitable inserting instrument, generally a surgical needle, while in the nonelongated or only minimally elongated state. Application of a pulling load between the two ends of the thread outside the tissue renders the anchoring structures, formed as barbs in the above examples, erectable or deployable. By subsequent gradual withdrawal of the pulling load, the inserted thread starts with a length reduction by closing the anchoring structures, causing these to anchor themselves in the tissue and at the same time to approximate and gather together the tissue, for example wound edges, or facilitating lifting for example.

## Claims

1. Elastomeric thread (100), more particularly for use as a knotless or self-fixating wound closure system, wherein the thread (100) includes on its surface anchoring structures (120) for anchoring in biological, more particularly human and/or animal, tissues, **characterized in that** the anchoring structures (120) absent an axial pulling load on the thread (100) essentially do not protrude from the thread surface, so that the thread (100) has an essentially smooth surface and the anchoring structures (120) are configured to be erectable on imposition of an axial pulling load on the thread (100).

2. Elastomeric thread (100) according to claim 1, **characterized in that** the anchoring structures (120) are configured to be reversibly erectable on imposition of an axial pulling load on the thread (100).

3. Elastomeric thread (100) according to claim 1 or 2, **characterized in that** the thread (100) is formed from a block copolymer, block terpolymer, segmented polymer or polymer blend.

4. Elastomeric thread (100) according to any preceding claim, **characterized in that** the thread (100) is formed from a thermoplastic elastomer.

5. Elastomeric thread (100) according to any preceding claim, **characterized in that** the thread (100) is formed from nonabsorbable elastomers selected from the group consisting of thermoplastic elastomers based on olefin, thermoplastic elastomers based on urethane, or thermoplastic polyurethanes, thermoplastic copolyesters, styrene block copolymers, thermoplastic copolyamides, silicone elastomers and mixtures thereof.

6. Elastomeric thread (100) according to any one of claims 1 to 4, **characterized in that** the thread (100) is formed from absorbable elastomers selected from the group consisting of polyhydroxybutyrates, block copolymers comprising glycolide, lactide, ε-caprolactone, trimethylene carbonate, p-dioxanone, 3-hydroxybutyrate and/or 4-hydroxybutyrate, and block terpolymers comprising glycolide, lactide, ε-caprolactone, trimethylene carbonate, p-dioxanone, 3-hydroxybutyrate and/or 4-hydroxybutyrate.

7. Elastomeric thread (100) according to any preceding claim, **characterized in that** the anchoring structures (120) are formed as cuts into the surface of the thread (100).

8. Elastomeric thread (100) according to any preceding claim, **characterized in that** the anchoring structures (120) on imposition of an axial pulling load on the thread (100) form a unidirectional or bidirectional arrangement on the surface of the thread (100).

9. Elastomeric thread (100) according to any one of claims 1 to 7, **characterized in that** the anchoring structures (120) on imposition of an axial pulling load on the thread (100) point for a first thread
portion in the direction of a remaining second thread portion and for the remaining second thread portion point in the direction of the first thread portion.

10. Elastomeric thread (100) according to any one of claims 1 to 7, **characterized in that** the anchoring structures (120) on imposition of an axial pulling load on the thread (100) have a periodically changing, more particularly alternating, orientation on the thread surface.

11. Thread (100) according to any preceding claim, **characterized in that** the anchoring structures (120) are coated with a preferably stiffening material, more particularly with a stiffening polymer.

12. Elastomeric thread (100) according to any preceding claim, **characterized in that** the thread is designed as a mass thread or as a hollow thread, in particular as a tubular thread, preferably as a tube.

13. Wound closure system, preferably in the manner of a surgical suture, comprising an elastomeric thread (100) according to any preceding claim.

14. Surgical kit of parts, comprising an elastomeric thread (100) according to any one of claims 1 to 12 or a wound closure system according to claim 13 and at least a surgical inserting instrument.

15. Process for producing an elastomeric thread (100) having anchoring structures (120) on its surface according to any one of claims 1 to 12, **characterized in that** anchoring structures (120) are formed on the surface of an elastomeric thread.

## Patentansprüche

1. Elastomerer Faden (100), insbesondere zur Verwendung als knotenloses bzw. selbstfixierendes Wundverschlusssystem, wobei der Faden (100) auf seiner Oberfläche Verankerungsstrukturen (120) zur Verankerung in biologischen, insbesondere menschlichen und/oder tierischen, Geweben aufweist,
**dadurch gekennzeichnet, dass**
die Verankerungsstrukturen (120) ohne eine axiale Zugbelastung des Fadens (100) im Wesentlichen nicht von der Fadenoberfläche abstehen, so dass der Faden (100) eine im Wesentlichen glatte Oberfläche besitzt, und die Verankerungsstrukturen (120) bei axialer Zugbelastung des Fadens (100) aufrichtbar ausgebildet sind.

2. Elastomerer Faden (100) nach Anspruch 1, **dadurch gekennzeichnet, dass** die Verankerungsstrukturen (120) bei axialer Zugbelastung des Fadens (100) reversibel aufrichtbar ausgebildet sind.

3. Elastomerer Faden (100) nach Anspruch 1 oder 2, **dadurch gekennzeichnet, dass** der Faden (100) aus einem Blockcopolymer, Blockterpolymer, segmentierten Polymer oder Polymerblend gebildet ist.

4. Elastomerer Faden (100) nach einem der vorhergehenden Ansprüche, **dadurch gekennzeichnet, dass** der Faden (100) aus einem thermoplastischen Elastomer gebildet ist.

5. Elastomerer Faden (100) nach einem der vorhergehenden Ansprüche, **dadurch gekennzeichnet, dass** der Faden (100) aus nicht resorbierbaren Elastomeren, ausgewählt aus der Gruppe bestehend aus thermoplastischen Elastomeren auf Olefinbasis, thermoplastischen Elastomeren auf Urethanbasis bzw. thermoplastischen Polyurethanen, thermoplastischen Copolyestern, Styrol-Blockcopolymeren, thermoplastischen Copolyamiden, Silikon-Elastomeren und Mischungen davon, gebildet ist.

6. Elastomerer Faden (100) nach einem der Ansprüche 1 bis 4, **dadurch gekennzeichnet, dass** der Faden (100) aus resorbierbaren Elastomeren, ausgewählt aus der Gruppe bestehend aus Polyhydroxybutyraten, Blockcopolymeren, umfassend Glykolid, Lactid, ε-Caprolacton, Trimethylencarbonat, p-Dioxanon, 3-Hydroxybutyrat und/oder 4-Hydroxybutyrat, und Blockterpolymeren, umfassend Glykolid, Lactid, ε-Caprolacton, Trimethylencarbonat, p-Dioxanon, 3-Hydroxybutyrat und/oder 4-Hydroxybutyrat, gebildet ist.

7. Elastomerer Faden (100) nach einem der vorhergehenden Ansprüche, **dadurch gekennzeichnet, dass** die Verankerungsstrukturen (120) als Einschnitte in die Oberfläche des Fadens (100) ausgebildet sind.

8. Elastomerer Faden (100) nach einem der vorhergehenden Ansprüche, **dadurch gekennzeichnet, dass** die Verankerungsstrukturen (120) bei axialer Zugbelastung des Fadens (100) eine unidirektionale oder bidirektionale Anordnung auf der Oberfläche des Fadens (100) ausbilden.

9. Elastomerer Faden (100) nach einem der Ansprüche 1 bis 7, **dadurch gekennzeichnet, dass** die Verankerungsstrukturen (120) bei axialer Zugbelastung des Fadens (100) für einen ersten Fadenabschnitt in Richtung eines übrigen zweiten Fadenabschnittes weisen und für den übrigen zweiten Fadenabschnitt in Richtung des ersten Fadenabschnittes weisen.

10. Elastomerer Faden (100) nach einem der Ansprüche 1 bis 7, **dadurch gekennzeichnet, dass** die Verankerungsstrukturen (120) bei axialer Zugbelastung des Fadens (100) eine sich periodisch ändernde, insbesondere alternierende, Ausrichtung auf der Fadenoberfläche besitzen.

11. Faden (100) nach einem der vorhergehenden Ansprüche, **dadurch gekennzeichnet, dass** die Verankerungsstrukturen (120) mit einem vorzugsweise versteifenden Material, insbesondere einem versteifenden Polymer, beschichtet sind.

12. Elastomerer Faden (100) nach einem der vorhergehenden Ansprüche, **dadurch gekennzeichnet, dass** der Faden in Form eines massiven Fadens vorliegt oder als Hohlfaden, insbesondere als schlauchartiger Faden, bevorzugt als Schlauch, gestaltet ist.

13. Wundverschlusssystem, vorzugsweise nach Art eines chirurgischen Nahtmaterials, umfassend einen elastomeren Faden (100) nach einem der vorhergehenden Ansprüche.

14. Chirurgisches Kit mit Teilen, umfassend einen elastomeren Faden (100) nach einem der Ansprüche 1 bis 12 oder ein Wundverschlusssystem nach Anspruch 13 und zumindest ein chirurgisches Einführinstrument.

15. Verfahren zur Herstellung eines elastomeren Fadens (100) mit Verankerungsstrukturen (120) auf seiner Oberfläche nach einem der Ansprüche 1 bis 12, **dadurch gekennzeichnet, dass** auf der Oberfläche eines elastomeren Fadens Verankerungsstrukturen (120) ausgebildet werden.

## Revendications

1. Fil élastomère (100), en particulier pour utilisation en tant que système de fermeture de plaie sans noeud ou auto-fixatrice, le fil (100) présentant sur sa surface des structures d'ancrage (120) pour ancrage dans des tissus biologiques, en particulier des tissus humains et/ou animaux,
**caractérisé en ce que**
les structures d'ancrage (120), en absence d'une force de traction axiale sur le fil (100), essentiellement ne dépassent pas de la surface du fil, de sorte que le fil (100) a une surface essentiellement lisse et les structures d'ancrage (120) sont configurées pour être dressées par une force de traction axiale sur le fil (100).

2. Fil élastomère (100) selon la revendication 1, **caractérisé en ce que** les structures d'ancrage (120) sont configurées pour être dressées réversiblement par une force de traction axiale sur le fil (100).

3. Fil élastomère (100) selon la revendication 1 ou 2, **caractérisé en ce que** le fil (100) est formé d'un copolymère en bloc, terpolymère en bloc, polymère segmenté ou mélange de polymère.

4. Fil élastomère (100) selon l'une quelconque des revendications précédentes, **caractérisé en ce que** le fil (100) est formé d'un élastomère thermoplastique.

5. Fil élastomère (100) selon l'une quelconque des revendications précédentes, **caractérisé en ce que** le fil (100) est formé d'élastomères non-résorbables, sélectionnés parmi le groupe constitué par élastomères thermoplastiques à base d'oléfine, élastomères thermoplastiques à base d'uréthane, ou polyuréthanes thermoplastiques, copolyesters thermoplastiques, copolymères en bloc de styrène, copolyamides thermoplastiques, élastomères de silicone et mélanges de ceux-ci.

6. Fil élastomère (100) selon l'une quelconque des revendications 1 à 4, **caractérisé en ce que** le fil (100) est formé d'élastomères résorbables, sélectionnés parmi le groupe constitué par polyhydroxybutyrates, copolymères en bloc comprenant glycolide, lactide, ε-caprolactone, carbonate de triméthylène, p-dioxanone, 3-hydroxybutyrate et/ou 4-hydroxybutyrate, et terpolymères en bloc comprenant glycolide, lactide, e-caprolactone, carbonate de triméthylène, p-dioxanone, 3-hydroxybutyrate et/ou 4-hydroxybutyrate.

7. Fil élastomère (100) selon l'une quelconque des revendications précédentes, **caractérisé en ce que** les structures d'ancrage (120) sont formées sous forme d'entailles dans la surface du fil (100).

8. Fil élastomère (100) selon l'une quelconque des revendications précédentes, **caractérisé en ce que**, par une force de traction axiale sur le fil (100), les structures d'ancrage (120) forment un arrangement unidirectionnel ou bidirectionnel sur la surface du fil (100).

9. Fil élastomère (100) selon l'une quelconque des revendications 1 à 7, **caractérisé en ce que**, par une force de traction axiale sur le fil (100), les structures d'ancrage (120) pointent pour une première partie du fil vers la direction d'une deuxième partie du fil restante et pointent pour la deuxième partie du fil restante vers la direction de la première partie du fil.

10. Fil élastomère (100) selon l'une quelconque des revendications 1 à 7, **caractérisé en ce que**, par une force de traction axiale sur le fil (100), les structures d'ancrage (120) présentent une orientation changeant périodiquement, en particulier alternant, sur la surface du fil.

11. Fil (100) selon l'une quelconque des revendications précédentes, **caractérisé en ce que** les structures d'ancrage (120) sont recouverts d'un matériau de préférence raidissant, en particulier d'un polymère raidissant.

12. Fil élastomère (100) selon l'une quelconque des revendications précédentes, **caractérisé en ce que** le fil est conçu sous forme d'un fil de masse ou d'un fil creux, en particulier sous forme d'un fil tubulaire, de préférence sous forme d'un tube.

13. Système de fermeture de plaie, de préférence de la manière d'un matériel de suture chirurgical, comprenant un fil élastomère (100) selon l'une quelconque des revendications précédentes.

14. Trousse chirurgicale de composants, comprenant un fil élastomère (100) selon l'une quelconque des revendications 1 à 12 ou un système de fermeture de plaie selon la revendication 13 et au moins un instrument chirurgical d'insertion.

15. Procédé de production d'un fil élastomère (100) présentant sur sa surface des structures d'ancrage (120) selon l'une quelconque des revendications 1 à 12, **caractérisé en ce que** des structures d'ancrage (120) sont formées sur la surface d'un fil élastomère.
